# EUROPEAN PATENT APPLICATION

(11) **EP 4 782 098 A1**
(43) Date of publication of application: **29.07.2026**
(21) Application number: 24877216.2
(22) Date of filing: 09.10.2024
(51) Int. Cl.: B01J 19/00, C12M 1/00, C12M 3/00, D01F 9/04

(54) **HYDROGEL MANUFACTURING APPARATUS AND HYDROGEL MANUFACTURING METHOD**

(30) Priority: 10.10.2023 JP 2023175600
(71) Applicant: Cellfiber Co., Ltd., Tokyo 135-0031 (JP)
(72) Inventor: IKEDA, Kazuhiro, Tokyo 135-0031 (JP)
(74) Representative: Isarpatent
(86) International application number: PCT/JP2024/036196
(87) International publication number: WO 2025/079621

(57) **Abstract**

Provided is a hydrogel production apparatus capable of suppressing contamination of impurities from formation of a hydrogel until usage thereof. A hydrogel production apparatus (100) comprises: a first flow path (212) for flowing a hydrogel precursor; a second flow path (222) for flowing a gelling agent that gels the hydrogel precursor; a first junction point (216) of the first flow path (212) and the second flow path (222); a storage container (400) containing a hydrogel that is formed by contact between the hydrogel precursor and the gelling agent; and a hydrogel flow path (300) that communicates in a closed manner at least between the storage container (400) and the first junction point (216).

## Description

### Technical Field

The present invention relates to a hydrogel production apparatus and a hydrogel production method.

### Background Art

The following Patent Literature 1 discloses a method for producing a hollow hydrogel fiber made of an alginate polymer. In the method described in Patent Literature 1, a hydrogel precursor is flowed along the core fluid around the core fluid, and furthermore, a gelling agent is flowed around the hydrogel precursor. As a result, an elongated fibrous hydrogel is formed by the hydrogel precursor undergoing gelation.

### Citation List

### Patent Literature

Patent Literature 1: WO 2011/046105 A1

### Summary

In Patent Literature 1, the hydrogel is formed in a container suitable for forming the hydrogel. The formed hydrogel is usually transferred to another storage container suitable for use when the hydrogel is used. Therefore, during the transfer of the hydrogel, there is a risk that impurities may be mixed into the hydrogel or into the solution containing the hydrogel.

Therefore, a hydrogel production apparatus and a hydrogel production method capable of suppressing the contamination of impurities from the formation of the hydrogel until the time of use are desired.

In one aspect, a hydrogel production apparatus comprises: a first flow path for flowing a hydrogel precursor; a second flow path for flowing a gelling agent that gels the hydrogel precursor; a first junction point of the first flow path and the second flow path; a storage container containing a hydrogel that is formed by contact between the hydrogel precursor and the gelling agent; and a hydrogel flow path that communicates in a closed manner at least between the storage container and the first junction point.

In one aspect, a hydrogel production method using the hydrogel production apparatus described above comprises: flowing the hydrogel precursor into the first flow path; flowing the gelling agent into the second flow path; joining the hydrogel precursor and the gelling agent at the first junction point to form a hydrogel; and introducing the hydrogel into the storage container through the hydrogel flow path.

### Brief Description of Drawings

Fig. 1 is a schematic diagram of a hydrogel production apparatus according to a first embodiment.
Fig. 2 is a schematic diagram for explaining the structure of a flow path of the hydrogel production apparatus according to the first embodiment.
Fig. 3 is a block diagram of the hydrogel production apparatus according to the first embodiment.
Fig. 4 is a perspective view showing an example of the structure of hydrogel formed in the first embodiment.
Fig. 5 is a cross-sectional view of the hydrogel shown in Fig. 4.
Fig. 6 is a diagram showing an example of data stored in a storage unit in the first embodiment.
Fig. 7 is a schematic diagram for explaining the state in each introduction path in an initial stage of a leak test.
Fig. 8 is a schematic diagram for explaining the state in each introduction path in a subsequent stage following Fig. 7.
Fig. 9 is a schematic diagram for explaining the state in each introduction path in the subsequent stage following Fig. 8.
Fig. 10 is a schematic diagram for explaining the state in each introduction path in a pre-liquid feeding (pre-feeding) stage.
Fig. 11 is a schematic diagram for explaining the state in each introduction path in the preparation stage of pulsation suppressing unit and/or defoaming unit.
Fig. 12 is a schematic diagram for explaining the state in each introduction path in the subsequent stage following Fig. 11.
Fig. 13 is a schematic diagram for explaining the state in each introduction path in the stage during the formation of the hydrogel.
Fig. 14 is a schematic diagram for explaining the structure of the flow path of the hydrogel production apparatus according to a second embodiment.
Fig. 15 is a block diagram of the hydrogel production apparatus according to the second embodiment.
Fig. 16 is a perspective view showing an example of the structure of the hydrogel formed in the second embodiment.
Fig. 17 is a cross-sectional view of the hydrogel shown in Fig. 16.
Fig. 18 is a diagram showing an example of data stored in a storage unit in the second embodiment.

### Description of Embodiments

Hereinafter, embodiments will be described with reference to the drawings. In the following drawings, the same or similar parts are denoted by the same or similar reference numerals. However, it should be noted that the drawings are schematic, and ratios of dimensions and the like may be different from actual ones.

### [First Embodiment]

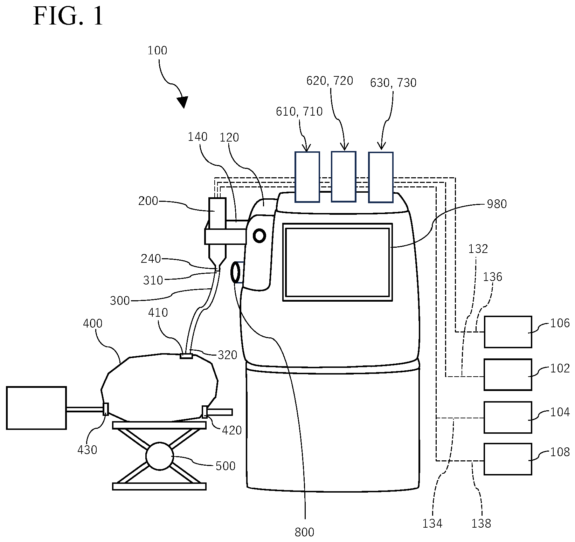

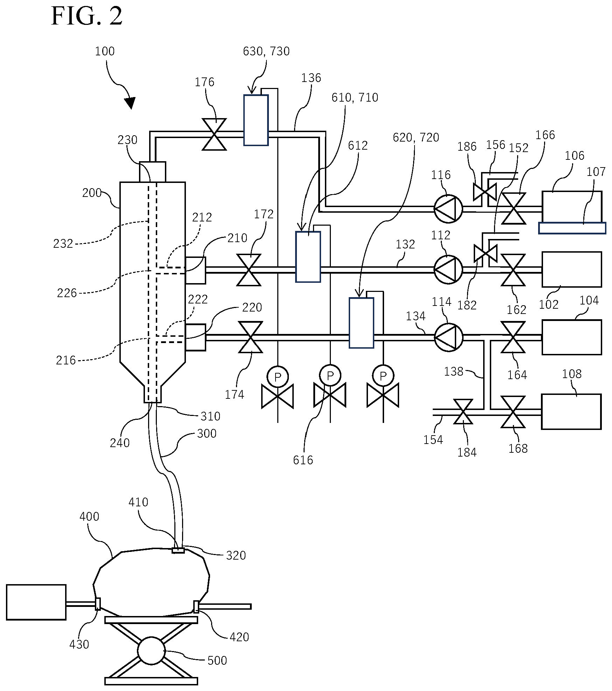

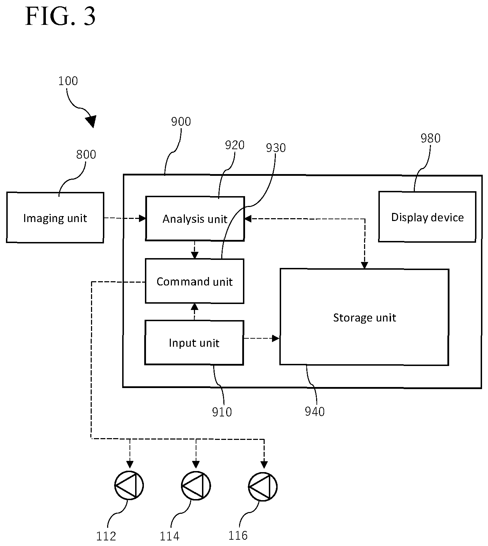

Fig. 1 is a schematic diagram of a hydrogel production apparatus according to a first embodiment. Fig. 2 is a schematic diagram for explaining the structure of a flow path of the hydrogel production apparatus according to the first embodiment. Fig. 3 is a block diagram of the hydrogel production apparatus according to the first embodiment. It should be noted that in Fig. 1, the structure of the flow path is either schematically shown or not shown. A hydrogel production apparatus 100 may include a first storage section 102, a second storage section 104, a third storage section 106, and a flow path forming member 200.

The first storage section 102 stores a first fluid. The first fluid is a hydrogel precursor (the same applies hereinafter). The hydrogel precursor is preferably a solution (including sol) containing, for example, alginate or agarose. More specifically, the first fluid may be a solution containing, for example, sodium alginate, potassium alginate, and ammonium alginate, or a combination thereof. The alginate may be a natural extract or may be chemically modified. Examples of the chemically modified alginate include methacrylate-modified alginate. In addition, the first fluid may be a mixed system of the abovementioned alginate and substances such as agar, agarose, polyethylene glycol (PEG), polylactic acid (PLA), or nanocellulose. The first fluid may contain an excipient and/or a thickener in addition to the hydrogel precursor.

The second storage section 104 stores the second fluid (gelling agent) that gelates the first fluid (hydrogel precursor) by coming into contact with the first fluid. When the first fluid is the alginate polymer material, the second fluid may be a solution containing polyvalent cations. Examples of such a solution include a solution containing calcium ions or barium ions, such as calcium chloride or barium chloride. The alginate polymer material is crosslinked by a polyvalent cation to form an alginate gel. The second fluid may contain an excipient and/or a thickener in addition to the gelling agent.

The third storage section 106 stores a third fluid. The third fluid may be a sol, a gel, or a liquid (including a suspension). Preferably, the third fluid may be a gel or a liquid. The type of gel or liquid constituting the third fluid is not particularly limited. These gels or liquids may include, for example, alginate solution, chitosan gel, collagen gel, gelatin, peptide gel, fibrin gel, laminin gel, nanocellulose, pullulan, dextran, culture medium, or mixtures thereof. The third fluid may contain an excipient and/or a thickener.

The hydrogel production apparatus 100 may have a second operation unit 107 that moves the fluid in the third storage section 106. When the fluid in the third storage section 106 contains suspended particles, the second operation unit 107 may be, for example, a stirrer that stirs the third fluid in the third storage section 106, a swing unit that swings the third storage section 106 itself, or the like.

When the third fluid contains, for example, suspended particles, the suspended particles in the third fluid in the third storage section 106 are uniformly dispersed by the action of the second operation unit 107. As a result, when a hydrogel is formed as described later, suspended particles are uniformly contained in the hydrogel.

The flow path forming member 200 is a member including a flow path through which the first fluid, the second fluid, and the third fluid flow. The flow path forming member 200 is preferably formed of an inflexible member. The flow path forming member 200 may be made of, for example, a member made of metal or synthetic resin.

The flow path forming member 200 may have a first inlet 210, a second inlet 220, a third inlet 230, and an outlet 240. The first inlet 210 is an inlet for the first fluid and is in fluid communication with the first storage section 102. The second inlet 220 is an inlet for the second fluid and is in fluid communication with the second storage section 104. The third inlet 230 is an inlet for the third fluid and is in fluid communication with the third storage section 106.

The flow path forming member 200 may have a first flow path 212, a second flow path 222, and a third flow path 232. The first flow path 212, the second flow path 222, and the third flow path 232 may be formed in the flow path forming member 200.

The first flow path 212 is a flow path through which the hydrogel precursor flows, and may be defined by a flow path from the first inlet 210 to the outlet 240 via a first junction point 216 described later. In the present embodiment, the first flow path 212 reaches the outlet 240 from the first inlet 210 via a second junction point 226 and a first junction point 216 described later. The first fluid reaches the outlet 240 from the first inlet 210 via the first flow path 212.

The second flow path 222 may be defined by a flow path from the second inlet 220 to the first junction point 216. The second flow path 222 is a flow path through which the gelling agent flows. The first junction point 216 is a junction point of the first flow path 212 and the second flow path 222. The second fluid is introduced from the second inlet 220 to the first flow path 212 via the second flow path 222 and the first junction point 216, and then reaches the outlet 240.

The third flow path 232 may be defined by a flow path from the third inlet 230 to the second junction point 226. The second junction point 226 is a junction point of the first flow path 212 and the third flow path 232. The second junction point 226 may be located on the upstream side of the first junction point 216 in the flow direction of the first flow path 212. That is, the third flow path 232 is configured to join the first flow path 212 at the second junction point 226 on the upstream side of the first junction point 216. The third fluid is introduced from the third inlet 230 to the first flow path 212 via the third flow path 232, the second junction point 226, and the first junction point 216, and then reaches the outlet 240.

The second flow path 222 is configured to join the second fluid (gelling agent) around the first fluid in the first flow path 212 at the first junction point 216. As a result, the second fluid flows along the flow direction of the first fluid around the flow of the first fluid. That is, the second fluid surrounds the first fluid (hydrogel precursor) in a cross section orthogonal to the flow of the first fluid.

When the first fluid (hydrogel precursor) and the second fluid (gelling agent) are brought into contact with each other at the first junction point 216, a hydrogel is formed. The hydrogel flows downstream from the first junction point 216 toward the outlet 240.

Preferably, the first fluid and the second fluid flow through the first flow path 212 and the second flow path 222 as laminar flows, respectively. As a result, the second fluid is hardly mixed with the first fluid and neatly surrounds the periphery of the first fluid in the cross section orthogonal to the flow direction of the first fluid. Therefore, a hydrogel having a well-formed shape is easily formed.

In the first embodiment, the first flow path 212 is configured to join the first fluid around the third fluid flowing into the first flow path 212 at the second junction point 226. As a result, the first fluid flows along the flow direction of the third fluid around the flow of the third fluid. That is, the first fluid surrounds the third fluid in a cross section orthogonal to the flow direction of the third fluid. That is, the first fluid flows while maintaining a tubular shape surrounding the third fluid, and then comes into contact with the second fluid at the first junction point 216 to form a hydrogel. Thereby, a hydrogel having a tubular shape can be formed.

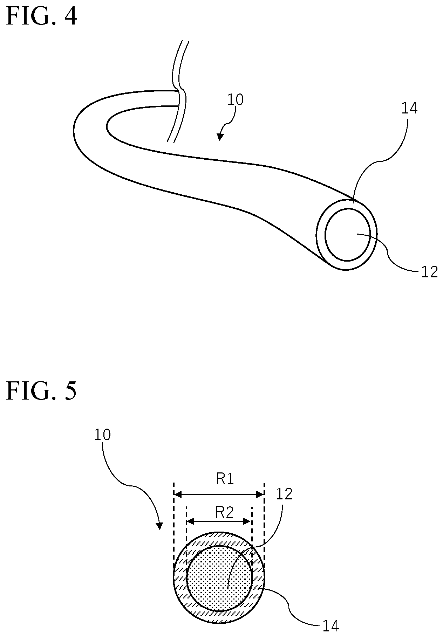

Preferably, the first fluid and the third fluid flow through the first flow path 212 and the third flow path 232 as laminar flows, respectively. As a result, the first fluid neatly surrounds the periphery of the third fluid with hardly mixing with the third fluid in the cross section orthogonal to the flow of the third fluid. Therefore, a tubular hydrogel having a well-formed shape is easily formed (see also Fig. 4).

As described above, the flow path forming member 200 is preferably formed of an inflexible member. In this case, since the first flow path 212, the second flow path 222, and the third flow path 232 are hardly deformed by an external force, a laminar flow including the first fluid, the second fluid, and/or the third fluid is easily stably formed in the first flow path 212, the second flow path 222, and the third flow path 232, respectively. Therefore, a hydrogel having a better-formed tubular shape is easily produced.

The sizes such as the flow path diameters of the first flow path 212, the second flow path 222, and the third flow path 232 can be appropriately designed according to the size and the like of each part of the hydrogel to be produced. In order to form an elongated or small hydrogel, the flow path diameters of the first flow path 212, the second flow path 222, and the third flow path 232 may be small. The flow path diameters (inner diameters) of the first flow path 212, the second flow path 222, and the third flow path 232 may be, for example, 5 mm or less or 3 mm or less. In addition, the flow path diameter (inner diameter) in the outlet 240 may be, for example, 30 mm or less, 10 mm or less, 5 mm or less, or 3 mm or less.

The distance from the first junction point 216 to the outlet 240 may be, for example, in a range of 3 mm to 150 mm, preferably in a range of 5 mm to 100 mm, and more preferably in a range of 10 mm to 50 mm. When the distance from the first junction point 216 to the outlet 240 is long, the shape of the hydrogel is stabilized in the flow path forming member 200, and then the hydrogel flows out from the outlet 240. Therefore, it is possible to suppress the collapse of the hydrogel in a hydrogel flow path 300. In addition, the shorter the distance from the first junction point 216 to the outlet 240, the smaller the size of the flow path forming member 200 can be.

The hydrogel production apparatus 100 may include a housing 120 and a support section 140 that supports the flow path forming member 200. The support section 140 is provided in the housing 120, and fixes the flow path forming member 200 to the housing 120. Accordingly, it is possible to suppress the vibration of the flow path forming member 200 during the production of the hydrogel.

The hydrogel production apparatus 100 may include: a first introduction path 132 that fluidly communicates the first storage section 102 with the first flow path 212 of the flow path forming member 200; a second introduction path 134 that fluidly communicates the second storage section 104 with the second flow path 222 of the flow path forming member 200; and a third introduction path 136 that fluidly communicates the third storage section 106 with the third flow path 232 of the flow path forming member 200. These introduction paths 132, 134, and 136 may be formed, for example, in an inflexible member or may be formed in a flexible tube. These introduction paths 132, 134, and 136 may be attached to the housing 120 or may be provided in the housing 120.

The hydrogel production apparatus 100 may include a first pump 112 that feeds a first fluid, a second pump 114 that feeds a second fluid, and a third pump 116 that feeds a third fluid. The first pump 112, the second pump 114, and the third pump 116 may be configured to be able to adjust flow rates and flow velocities of the first fluid, the second fluid, and the third fluid, respectively.

The flow rate settings in the first pump 112, the second pump 114, and the third pump 116 may be configured to be adjustable by the user, or may be automatically adjusted by a preset program.

The types of the first pump 112, the second pump 114, and the third pump 116 are not particularly limited. Preferably, the first pump 112, the second pump 114, and the third pump 116 may be pumps that reduce pulsation of the fed liquid. The first pump 112, the second pump 114, and the third pump 116 may be pumps having performance such that, excluding periods of startup and shutdown of the pump, at a specific average flow velocity value, for example, within the range of 0.1 mL to 1000 mL/min, the variation in flow velocity remains within, for example, 20%, preferably within 10%, more preferably within 5%, and even more preferably within 2% of the specific average flow velocity value.

The first introduction path 132 may include a first introduction valve 162 between the first storage section 102 and first pulsation suppressing unit 610 and/or first defoaming unit 710 described later. When the first pump 112 is operated in a state where the first introduction valve 162 is opened, the first fluid flows from the first storage section 102 toward the first introduction path 132. The flow path cross-sectional area of the first introduction path 132 is preferably larger than the flow path cross-sectional area of the second introduction path 134 and/or the third introduction path 136. The first fluid flowing through the first introduction path 132 is a hydrogel precursor and has a relatively high viscosity. Therefore, when the flow path cross-sectional area of the first introduction path 132 is large, it is easy to flow the first fluid having high viscosity without excessively increasing the pump pressure of first pump 112. From such a viewpoint, the first introduction path 132 may be branched into a plurality of paths in parallel instead of one path. In this case, the flow path cross-sectional area of the first introduction path 132 is defined by the sum of the cross-sectional areas of the plurality of flow paths.

The second introduction path 134 may include a second introduction valve 164 between the second storage section 104 and second pulsation suppressing unit 620 and/or second defoaming unit 720 described later. When the second pump 114 is operated in a state where the second introduction valve 164 is opened, the second fluid flows from the second storage section 104 toward the second introduction path 134.

The third introduction path 136 may include a third introduction valve 166 between the third storage section 106 and third pulsation suppressing unit 630 and/or third defoaming unit 730 described later. When the third pump 116 is operated in a state where the third introduction valve 166 is opened, the third fluid flows from the third storage section 106 toward the third introduction path 136.

The hydrogel production apparatus 100 may have a cleaning mechanism that cleans at least a part of the first flow path 212, the second flow path 222, and the third flow path 232. In the first embodiment, the hydrogel production apparatus 100 may have a cleaning container 108 for storing a cleaning liquid. The cleaning liquid is preferably water, pure water, physiological saline, or the like. The second pump 114 may also serve as a pump not only for feeding the second fluid but also for feeding the cleaning liquid. Alternatively, a dedicated pump (cleaning pump) for feeding the cleaning liquid may be provided. In this case, the cleaning pump may be provided in the cleaning flow path 138 through which substantially only the cleaning liquid flows.

The cleaning container 108 is preferably in fluid communication with at least one of the first flow path 212, the second flow path 222, and the third flow path 232 via the cleaning flow path 138. In the first embodiment, the cleaning container 108 is joined to the second introduction path 134. As a result, the cleaning liquid can reach the outlet 240 through the cleaning flow path 138, the second introduction path 134, the second flow path 222, and the first junction point 216. Alternatively, the cleaning container 108 may be joined to the first introduction path 132. In addition, the cleaning container 108 may be joined to both the first introduction path 132 and the second introduction path 134. In this case, the cleaning container 108 and the cleaning flow path 138 connected to the first introduction path 132 and the cleaning container 108 and the cleaning flow path 138 connected to the second introduction path 134 may be separately provided.

The cleaning flow path 138 may have a cleaning valve 168. When the cleaning pump and/or the second pump 114 is operated in a state where the cleaning valve 168 is opened, the cleaning liquid flows from the cleaning container 108 toward the cleaning flow path 138 and the second introduction path 134.

The hydrogel production apparatus 100 may include a first air introduction path 152, a second air introduction path 154, and a third air introduction path 156 for introducing gas into each of the first introduction path 132, the second introduction path 134, and the third introduction path 136. The first air introduction path 152, the second air introduction path 154, and the third air introduction path 156 are connected to the first introduction path 132, the second introduction path 134, and the third introduction path 136, respectively. The first air introduction path 152, the second air introduction path 154, and the third air introduction path 156 are used to inspect leakage of the first introduction path 132, the second introduction path 134, and the third introduction path 136.

Each of the first introduction path 132, the second introduction path 134, and the third introduction path 136 may have air valves 182, 184, and 186. Whether or not gas is introduced into the first introduction path 132, the second introduction path 134, and the third introduction path 136 can be controlled by opening and closing the air valves 182, 184, and 186.

Here, by selectively opening the first introduction valve 162 and the air valve 182 for air, it is possible to selectively determine whether to introduce the first fluid into the first introduction path 132 or to introduce the gas into the first introduction path 132. Similarly, by selectively opening the third introduction valve 166 and the air valve 186, it is possible to selectively determine whether to introduce the third fluid into the third introduction path 136 or to introduce the gas into the third introduction path 136.

Further, by selectively opening the second introduction valve 164, the cleaning valve 168, and the air valve 184, it is possible to selectively determine whether to introduce the second fluid into the second introduction path 134, introduce the cleaning liquid into the second introduction path 134, or introduce the gas into the second introduction path 134.

The first introduction path 132 may include a fourth introduction valve 172 on the downstream side of the junction point of the first introduction path 132 and the first air introduction path 152. The second introduction path 134 may include a fifth introduction valve 174 on the downstream side of the junction point of the second introduction path 134 and the second air introduction path 154. The third introduction path 136 may include a sixth introduction valve 176 on the downstream side of the junction point of the third introduction path 136 and the third air introduction path 156.

Preferably, the fourth introduction valve 172 may be provided between the flow path forming member 200 and the first pulsation suppressing unit 610 and/or the first defoaming unit 710 described later. Preferably, the fifth introduction valve 174 may be provided between the flow path forming member 200 and the second pulsation suppressing unit 620 and/or the second defoaming unit 720 described later. Preferably, the sixth introduction valve 176 may be provided between the flow path forming member 200 and the third pulsation suppressing unit 630 and/or the third defoaming unit 730 described later.

The fourth introduction valve 172, the fifth introduction valve 174, and the sixth introduction valve 176 can be used to prevent the fluid from flowing back to the other introduction path through the flow path forming member 200 when the fluid flows to any one of the first introduction path 132, the second introduction path 134, and the third introduction path 136.

The hydrogel production apparatus 100 may include the hydrogel flow path 300 and a storage container 400. The storage container 400 is a container that contains hydrogel formed through the contact between the first fluid (hydrogel precursor) and the second fluid (gelling agent). The storage container 400 is not particularly limited, but may be a container suitable for the use environment and/or the use purpose of the hydrogel formed by the flow path forming member 200, more specifically, a container designed in advance according to the use environment and/or the use purpose.

The hydrogel flow path 300 is a flow path that communicates in a closed manner at least between the storage container 400 and the first junction point 216. Preferably, the hydrogel flow path 300 communicates in a closed manner between the outlet 240 of the flow path forming member 200 and the storage container 400. Here, the hydrogel flow path 300 is preferably configured to communicate from the first junction point 216 to the storage container 400 in an airtight and/or liquid-tight manner. As a result, the hydrogel formed through the contact between the first fluid (hydrogel precursor) and the second fluid (gelling agent) can be transferred to the storage container 400 away from the flow path forming member 200 in an airtight and/or liquid-tight state. Therefore, it is possible to suppress mixing of impurities into the formed hydrogel or the fluid flowing together with the hydrogel.

The hydrogel flow path 300 may have a first end portion 310 attachable to the part of the outlet 240 of the flow path forming member 200 and a second end portion 320 attachable to and detachable from the part of an inlet 410 of the storage container 400. As a result, the storage container 400 having a different design according to the use environment and/or the use purpose of the hydrogel can easily communicate with the outlet 240 of the flow path forming member 200.

The hydrogel flow path 300 is not particularly limited, but may have a length of, for example, 0.1 m to 10 m, 0.2 m to 8 m, or 0.4 m to 5 m. As a result, the hydrogel can be transferred from the flow path forming member 200 to the storage container 400 relatively far away in a closed system state.

The hydrogel flow path 300 may be constituted by a flexible tube or a rigid tube. When the hydrogel flow path 300 is constituted by a rigid tube, the shape of the hydrogel flow path 300 is easily maintained stably.

On the other hand, in the case where the hydrogel flow path 300 is constituted by a flexible tube, even when the positional relationship between the flow path forming member 200 and the storage container 400 changes, the hydrogel flow path 300 can maintain a state where the hydrogel flow path 300 communicates in a closed manner between the storage container 400 and the first junction point 216 by the bending of the tube. When the storage containers 400 having different designs according to the use environment and/or the use purpose of the hydrogel are used, the installation position of the storage container 400 may be restricted. Even when the installation position of the storage container 400 is changed due to the constraint, the hydrogel flow path 300 constituted by a flexible tube can maintain a state where the hydrogel flow path 300 communicates in a closed manner between the storage container 400 and the first junction point 216.

The inner diameter (diameter of the internal space) of the hydrogel flow path 300 is not particularly limited, but may be, for example, 1 to 400 times, preferably 1 to 100 times, more preferably 1 to 40 times, and still more preferably 1 to 10 times the flow path diameter (inner diameter) in the outlet 240 of the flow path forming member 200. Since the hydrogel formed in the flow path forming member 200 passes through, the inner diameter of the hydrogel flow path 300 is desirably 1 time or more the inner diameter of the outlet 240. In addition, in order to suppress bending of the tube-shaped hydrogel formed in the flow path forming member 200 in the hydrogel flow path 300, the upper limit of the hydrogel flow path 300 is desirably equal to or less than the above desired value.

In the shown aspect, the hydrogel flow path 300 is constituted by a single tube. Alternatively, the hydrogel flow path 300 may be configured by connecting a plurality of tubes in series.

The storage container 400 is not particularly limited as long as the storage container 400 is a container suitable for a use environment and/or a use purpose of the hydrogel. Preferably, the storage container 400 is constituted by a flexible container having an expandable and contractible internal space.

When the internal space of the storage container 400 is neither expandable nor contractible, it is desirable to exhaust gas in the internal space of the storage container 400 in order to suppress an increase in pressure of the internal space of the storage container 400 when the hydrogel formed by the flow path forming member 200 is caused to flow into the storage container 400.

On the other hand, when the storage container 400 is constituted by a flexible container having an expandable and contractible internal space, the hydrogel can be caused to flow into the storage container 400 in a state where the internal space of the storage container 400 is in a depressed state. In this case, it is not necessary to exhaust the gas in the internal space of the storage container 400 while the hydrogel formed by the flow path forming member 200 is flowing into the storage container 400. Therefore, the possibility of contamination of impurities into the storage container 400 can be further reduced.

The hydrogel production apparatus 100 may have a first operation unit 500 that moves the liquid in the storage container 400. The first operation unit 500 may be, for example, a stirrer that stirs the liquid in the storage container 400, a swing unit that swings the storage container 400 itself, or the like.

Even when the first operation unit 500 is a swing unit that swings the storage container 400 itself, if the hydrogel flow path 300 is constituted by a flexible tube, there is an advantage in that the influence of the swinging of the storage container 400 is less likely to be transmitted to the flow path forming member 200.

The storage container 400 may have a discharge port 420 for discharging the liquid in the internal space and an introduction port 430 for introducing a liquid different from the liquid used at the time of forming the hydrogel into the internal space. By using the discharge port 420 and the introduction port 430, it is possible to remove and/or replace the liquid accumulated in the internal space of the storage container 400 at the time of forming the hydrogel.

The first fluid and/or the third fluid may contain cells as suspended particles. When the third fluid contains cells, the cells can be contained in the space inside the tubular hydrogel formed. When the first fluid contains cells, the cells are embedded in the tubular hydrogel formed. In these cases, the storage container 400 may be, for example, a culture container for culturing cells. In this case, the introduction port 430 may be a port for introducing the culture medium into the storage container 400.

The type of the cell is not particularly limited. Such cells may include, for example, pluripotent cells such as ES cells and iPS cells; various multipotent stem cells such as hematopoietic stem cells, neural stem cells, and mesenchymal stem cells; and unipotent stem cells such as hepatic stem cells and germline stem cells. Alternatively, the cells contained in the first fluid may be various differentiated cells, such as muscle cells including skeletal muscle cells and cardiomyocytes; nerve cells such as cerebral cortex cells; fibroblasts; epithelial cells; hepatocytes; pancreatic β cells; skin cells; and the like. Furthermore, it should be noted that the "cell" is not limited to a single cell, and includes a microorganism such as a bacterium in addition to a cellular tissue composed of a plurality of cells.

The first fluid and/or the third fluid may contain various growth factors suitable for cell culture, maintenance or proliferation of cells, or expression of cell functions, such as epidermal growth factor (EGF), platelet-derived growth factor (PDGF), transforming growth factor (TGF), insulin-like growth factor (IGF), fibroblast growth factor (FGF), and nerve growth factor (NGF).

The hydrogel production apparatus 100 may include the first pulsation suppressing unit 610, the second pulsation suppressing unit 620, and/or the third pulsation suppressing unit 630. The first pulsation suppressing unit 610 is a unit for suppressing pulsation of the first fluid generated by the first pump 112. The second pulsation suppressing unit 620 is a unit for suppressing pulsation of the second fluid generated by the second pump 114. The third pulsation suppressing unit 630 is a unit for suppressing pulsation of the third fluid generated by the third pump 116.

The first pulsation suppressing unit 610 may be provided on the upstream side of the first junction point 216, preferably on the upstream side of the second junction point 226, and more preferably on the upstream side of the flow path forming member 200. The second pulsation suppressing unit 620 may be provided on the upstream side of the first junction point 216, and preferably on the upstream side of the flow path forming member 200. The third pulsation suppressing unit 630 may be provided on the upstream side of the second junction point 226, and preferably on the upstream side of the flow path forming member 200.

The pulsation suppressing unit 610, 620, and 630 may include any units for suppressing pulsation. Since pulsation of the first fluid, the second fluid, and/or the third fluid is suppressed, each of the first fluid, the second fluid, and/or the third fluid hardly mixes with other fluids at the first junction point 216 and/or the second junction point 226, and a clean laminar flow is easily formed. Accordingly, it is possible to suppress variations in the shape of the hydrogel finally formed.

The hydrogel production apparatus 100 may include the first defoaming unit 710, the second defoaming unit 720, and/or the third defoaming unit 730. The first defoaming unit 710 is a unit for removing gas (bubbles) in the first fluid. The second defoaming unit 720 is a unit for removing gas (bubbles) in the second fluid. The third defoaming unit 730 is a unit for removing gas (bubbles) in the third fluid.

The first defoaming unit 710 may be provided on the upstream side of the first junction point 216, preferably on the upstream side of the second junction point 226, and more preferably on the upstream side of the flow path forming member 200. The second defoaming unit 720 may be provided on the upstream side of the first junction point 216, and preferably on the upstream side of the flow path forming member 200. The third defoaming unit 730 may be provided on the upstream side of the second junction point 226, and preferably on the upstream side of the flow path forming member 200.

By removing the bubbles in the first fluid, the second fluid, and/or the third fluid, each of the first fluid, the second fluid, and/or the third fluid easily forms a clean laminar flow at the first junction point 216 and/or the second junction point 226. Accordingly, it is possible to suppress variations in the shape of the hydrogel finally formed.

The first pulsation suppressing unit 610 and the first defoaming unit 710 may be separate from each other, or may be the same unit. When the first pulsation suppressing unit 610 and the first defoaming unit 710 are the same unit, both suppression of pulsation of the first fluid and removal of bubbles in the first fluid can be performed by one means (unit). Similarly, the second pulsation suppressing unit 620 and the second defoaming unit 720 may be separate unit from each other, or may be the same unit. In addition, the third pulsation suppressing unit 630 and the third defoaming unit 730 may be separate unit from each other, or may be the same unit.

In the shown aspect, the first pulsation suppressing unit 610 and the first defoaming unit 710 are configured by the same unit, that is, a common unit. Similarly, the second pulsation suppressing unit 620 and the second defoaming unit 720 are configured by the same unit, that is, a common unit. The third pulsation suppressing unit 630 and the third defoaming unit 730 are configured by the same unit, that is, a common unit.

In the shown example, the first pulsation suppressing unit 610 and the first defoaming unit 710 are located between the first pump 112 and the first junction point 216, and preferably between the first pump 112 and the flow path forming member 200. The first pulsation suppressing unit 610 and the first defoaming unit 710 have a storage section 612 capable of holding gas.

The storage section 612 communicates with the first introduction path 132 and may be sealed at a place other than the first introduction path 132. The first fluid sent from the first pump 112 to the storage section 612 via the first introduction path 132 is temporarily stored in the storage section 612. The air bubbles in the first fluid temporarily stored in the storage section 612 accumulate in the storage section 612. The first introduction path 132 communicates with the storage section 612 at a lower part of the storage section 612. Therefore, the air bubbles contained in the storage section 612 are less likely to be mixed into the flow path forming member 200.

Furthermore, when the first fluid accumulates in the storage section 612 and the pressure in the storage section 612 increases, the first fluid is fed from the storage section 612 toward the flow path forming member 200. Since the pressure fluctuation in the storage section 612 is smaller than the pulsation by the first pump 112, the first fluid is fed toward the flow path forming member 200 in a state where the pulsation of the first fluid caused by the influence of the first pump 112 is suppressed.

The first pulsation suppressing unit 610 and the first defoaming unit 710 preferably include a pressure sensor 616 that measures the pressure in the storage section 612. When the fourth introduction valve 172 is opened after the pressure detected by the pressure sensor 616 reaches a desired value, the first fluid can be appropriately fed toward the flow path forming member 200.

In the shown example, the second pulsation suppressing unit 620 and the second defoaming unit 720 are located between the second pump 114 and the first junction point 216, and preferably between the second pump 114 and the flow path forming member 200. Since the structures of the second pulsation suppressing unit 620 and the second defoaming unit 720 are similar to those of the first pulsation suppressing unit 610 and the first defoaming unit 710, the description thereof will be omitted.

In the shown example, the third pulsation suppressing unit 630 and the third defoaming unit 730 are located between the third pump 116 and the second junction point 226, and preferably between the third pump 116 and the flow path forming member 200. Since the structures of the third pulsation suppressing unit 630 and the third defoaming unit 730 are similar to those of the first pulsation suppressing unit 610 and the first defoaming unit 710, the description thereof will be omitted.

The hydrogel production apparatus 100 may have an imaging unit 800. The imaging unit 800 may be configured to acquire the form of the hydrogel on the downstream side of the first junction point 216 or at the first junction point 216. Preferably, the imaging unit 800 may be configured to acquire the form of the hydrogel on the downstream side of the outlet 240 of the flow path forming member 200, that is, in the hydrogel flow path 300.

The imaging unit 800 acquires the form of the hydrogel as an image (including a still image or a moving image). The image acquired by the imaging unit 800 is preferably sent to a control device 900 described later.

Fig. 4 is a perspective view showing an example of the structure of hydrogel formed in the first embodiment. Fig. 5 is a cross-sectional view of the hydrogel shown in Fig. 4. As shown in Fig. 4, the hydrogel 10 is formed in a long tubular shape. Hereinafter, the tubular hydrogel may be referred to as a "shell 14". In addition, an inner part of the tubular hydrogel, that is, a part formed by the first fluid may be referred to as a "core 12".

In a case where the storage container 400 is configured by a flexible container having an expandable and contractible internal space, it is preferable that the internal space of the storage container 400 is in an expandable state as described above before feeding the first fluid, the second fluid, and the third fluid at the time of producing the hydrogel.

The hydrogel production apparatus 100 may have the control device 900 that controls each component constituting the apparatus. The control device 900 may include an input unit 910, an analysis unit 920, a command unit 930, and a storage unit 940.

For example, the control device 900 may control the first pump 112, the second pump 114, and the third pump 116, and may control opening and closing of the first introduction valve 162, the second introduction valve 164, the third introduction valve 166, the fourth introduction valve 172, the fifth introduction valve 174, the sixth introduction valve 176, the cleaning valve 168, and/or the air valve 182, 184, and 186.

The control device 900 may include a central processing unit (CPU), a read only memory (ROM), and a random access memory (RAM). The CPU is an arithmetic device. An example of details of the control by the control device 900 will be described in detail below. The ROM stores programs and the like for realizing various processes by the CPU. Such a program may be, for example, a program for executing various types of control described in the above-described embodiments. The RAM stores data necessary for various processes by the CPU.

The input unit 910 receives an input by a user who uses the hydrogel production apparatus 100. The input unit 910 receives an input from an input device such as a keyboard, a mouse, or a touch panel.

In one example, the input unit 910 may be configured to be able to receive respective set values of the flow velocity in the first flow path, the flow velocity in the second flow path, and the flow velocity in the third flow path. In other words, the user can input each of the flow velocity of the first fluid, the flow velocity of the second fluid, and the flow velocity of the third fluid from the input device.

Preferably, the input unit 910 is configured to be able to receive each of the flow velocity of the first flow path, the flow velocity of the second flow path, and the flow velocity of the third flow path. Alternatively, the input unit 910 may be configured to be able to receive any one or two of the set values of the flow velocity of the first flow path, the flow velocity of the second flow path, and the flow velocity of the third flow path.

The command unit 930 sends a command to operate the first pump 112, the second pump 114, and the third pump 116. When receiving the set values of the flow velocity of the first fluid, the flow velocity of the second fluid, and the flow velocity of the third fluid from the input unit 910, the command unit 930 operates the first pump 112, the second pump 114, and the third pump 116 based on the input set values of the flow velocity. As a result, it is possible to set and/or change the flow velocity of the first fluid, the flow velocity of the second fluid, and the flow velocity of the third fluid according to the request for the set value of the flow velocity from the user.

Here, the flow velocity of the first fluid, the flow velocity of the second fluid, and the flow velocity of the third fluid affect values related to the size of the tubular hydrogel to be formed. Specifically, the flow velocity V1 of the first fluid, the flow velocity V2 of the second fluid, and the flow velocity V3 of the third fluid affect the diameter R1 of the shell (the diameter of the entire hydrogel), the diameter R2 of the core, and the ratio (S3/S1) of the cross-sectional area S3 of the core to the cross-sectional area S1 of the shell in the tubular hydrogel to be formed (see also Fig. 5). Here, the cross-sectional area is an area in a cross section in a direction orthogonal to the longitudinal direction of the tube. For example, the flow velocity of the first fluid, the flow velocity of the second fluid, and the flow velocity of the third fluid have a relationship between the diameter R1 of the shell, the diameter R2 of the core, and the ratio (S3/S1) of the cross-sectional area S3 of the core to the cross-sectional area S1 of the shell in the tubular hydrogel as shown in Table 1 below. It should be noted that in the following Table 1, "Reference" is merely a reference for comparison with Examples 1 to 3.

**(Table 1)**

| | Flow velocity of First fluid (V1) | Flow velocity of Second fluid (V2) | Flow velocity of Third fluid (V3) | Diameter of Shell (R1) | Diameter of Core (R2) | Ratio of cross-sectional area of Core to cross-sectional area of Shell (S3 / S1) |
|---|---|---|---|---|---|---|
| Reference | middle | middle | middle | middle | middle | middle |
| Example 1 | middle | high | middle | small | small | middle |
| Example 2 | high | middle | middle | large | middle | small |
| Example 3 | middle | middle | high | large | large | large |

More specifically, when the flow velocity of the second fluid is increased without changing the flow velocities of the first fluid and the third fluid, both the diameter R1 of the shell and the diameter R2 of the core are decreased (comparison between the Reference and Example 1 in Table 1). However, the ratio (S3/S1) of the cross-sectional area S3 of the core to the cross-sectional area S1 of the shell does not change much. In addition, when the flow velocity of the first fluid is increased without changing the flow velocities of the second fluid and the third fluid, the diameter R1 of the shell becomes large, and the ratio (S3/S1) of the cross-sectional area S3 of the core to the cross-sectional area S1 of the shell becomes small (comparison between the Reference and Example 2 in Table 1). Furthermore, when the flow velocity of the third fluid is increased without changing the flow velocities of the first fluid and the second fluid, the diameter R1 of the shell and the diameter R2 of the core are increased, and the ratio (S3/S1) between the cross-sectional area S3 of the core and the cross-sectional area S1 of the shell is also increased (comparison between the Reference and Example 3 in Table 1).

Therefore, the user can appropriately adjust the diameter R1 of the shell, the diameter R2 of the core, and the ratio (S3/S1) of the cross-sectional area S3 of the core to the cross-sectional area S1 of the shell in the tubular hydrogel to be formed by appropriately setting the flow velocity V1 of the first fluid, the flow velocity V2 of the second fluid, and the flow velocity V3 of the third fluid.

The control device 900 may acquire image data of the hydrogel obtained by the imaging device 800. The analysis unit 920 may be configured to analyze the form of the hydrogel imaged by the imaging unit 800.

The analysis unit 920 analyzes the image of the hydrogel captured in the image by known image analysis processing. Preferably, the analysis unit 920 calculates a value related to the size of the formed hydrogel. Specifically, the analysis unit 920 preferably calculates the diameter R1 of the shell, the diameter R2 of the core, and/or the ratio (S3/S1) of the cross-sectional area S3 of the core to the cross-sectional area S1 of the shell in the formed hydrogel.

The storage unit 940 may store the results of the analysis by the analysis unit 920 together with the set value of the flow velocity set by the input unit 910. The value related to the size of the hydrogel calculated by the analysis unit 920 is preferably stored in the storage unit 940 in association with information related to the flow velocity V1 of the first fluid, the flow velocity V2 of the second fluid, and the flow velocity V3 of the third fluid at the time of producing the hydrogel.

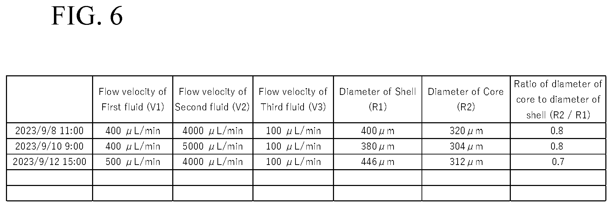

Fig. 6 is a diagram showing an example of data stored in the storage unit in the first embodiment. As shown in Fig. 6, the diameter R1 of the shell, the diameter R2 of the core, and the ratio (S3/S1) of the cross-sectional area S3 of the core to the cross-sectional area S1 of the shell in the hydrogel calculated by the analysis unit 920 may be stored in the storage unit 940 in association with the flow velocity V1 of the first fluid, the flow velocity V2 of the second fluid, and the flow velocity V3 of the third fluid at the time of producing the hydrogel.

The information on the size and the flow velocity of the hydrogel stored in the storage unit 940 may be configured to be able to be confirmed by the user according to a command from the user. For example, the information shown in Fig. 6 may be displayable on a display device 980 such as a monitor provided in the hydrogel production apparatus 100. In this case, the user can easily set an appropriate value of the flow velocity based on the data of the size of the hydrogel formed in the past at the time of newly producing the hydrogel.

In the above description, the case where the input unit 910 receives the flow velocity in the first flow path, the flow velocity in the second flow path, and the flow velocity in the third flow path has been described. However, the input unit 910 may be configured to be able to receive information on the size of the hydrogel to be formed. For example, the input unit 910 may be configured to be able to receive the diameter R1 of the shell, the diameter R2 of the core, and the like in the hydrogel to be formed.

When the control device 900 receives the information on the size of the hydrogel to be formed input by the user, for example, the diameter R1 of the shell or the diameter R2 of the core, the control device 900 automatically determines the flow velocity V1 of the first fluid, the flow velocity V2 of the second fluid, and the flow velocity V3 of the third fluid based on the information on the size of the hydrogel. The flow velocity V1 of the first fluid, the flow velocity V2 of the second fluid, and the flow velocity V3 of the third fluid can be acquired from, for example, information on the size of the hydrogel produced in the past. Specifically, the control device 900 can determine the flow velocity V1 of the first fluid, the flow velocity V2 of the second fluid, and the flow velocity V3 of the third fluid based on the past data (see Fig. 6) stored in the storage unit 940, that is, the relationship between the flow velocity V1 of the first fluid, the flow velocity V2 of the second fluid, the flow velocity V3 of the third fluid, and the size (diameters R1 and R2) of the hydrogel.

In this case, the command unit 930 of the control device 900 may control the flow velocities of the first fluid, the second fluid, and the third fluid according to the determined flow velocities V1, V2, and V3, that is, may control the first pump 112, the second pump 114, and the third pump 116.

Furthermore, the control device 900 may be configured to control (feedback control) the flow velocity of at least one of the first fluid (hydrogel precursor), the second fluid (gelling agent), and the third fluid based on the results of the analysis by the analysis unit 920. Specifically, as described above, the analysis unit 920 calculates information on the size of the hydrogel actually formed (for example, various diameters R1 and R2) based on the image of the hydrogel formed. Further, the analysis unit 920 calculates a deviation of the information (for example, various diameters R1 and R2) on the size of the hydrogel actually formed from the information on the size of the hydrogel received by the input unit 910. The command unit 930 may control, that is, change the flow velocity of at least one of the first fluid (hydrogel precursor), the second fluid (gelling agent), and the third fluid to eliminate the deviation.

As described above, the flow velocities of the first fluid, the second fluid, and the third fluid are related to the diameter R1 of the shell, the diameter R2 of the core, and/or the ratio (S3/S1) of the cross-sectional area S3 of the core to the cross-sectional area S1 of the shell as shown in Table 1. Based on this relationship, the control device 900 may change the flow velocity of at least one of the first fluid, the second fluid, and the third fluid such that the size of the hydrogel actually formed becomes close to the set value of the size of the hydrogel input by the user.

Instead of the above analysis, or in addition to the above analysis, the analysis unit 920 may be configured to estimate foreign matter in the formed hydrogel, such as cell density, based on image information captured by the imaging unit 800. The image information used for estimating the density of cells may be, for example, optical information and/or transparency information. In one example, instead of the above analysis or in addition to the above analysis, the analysis unit 920 may be configured to calculate the transparency of the image captured by the imaging unit 800 and to estimate foreign matter in the hydrogel, such as cell density, based on the transparency. Specifically, when the third fluid contains cells, the analysis unit 920 calculates the transparency of the core in the hydrogel in the image captured by the imaging unit 800. The analysis unit 920 estimates the cell density in the core of the hydrogel based on the transparency of the core.

When the third fluid contains cells, the transparency of the third fluid or the core is closely related to the density of the cells in the third fluid. That is, the higher the density of cells in the third fluid, the lower the transparency of the third fluid or the core. Therefore, the analysis unit 920 can estimate the cell density in the core of the hydrogel based on the transparency of the core in the formed hydrogel. The relationship between the transparency of the core and the cell density may be determined in advance by experiment. As a result, the hydrogel production apparatus 100 can quantitatively measure and store the density of cells mixed in the hydrogel, for example, in the use of cell culture experiments.

The analysis unit 920 may be configured to detect an abnormality in at least one of the hydrogel, the first flow path 212, and the second flow path 222 by analyzing an image (image information) captured by the imaging unit 800. The control device 900 may be configured to stop feeding at least one, preferably all, of the first fluid (hydrogel precursor), the second fluid (gelling agent), and the third fluid when the abnormality is detected.

Furthermore, the analysis unit 920 may be configured to detect abnormality of the third flow path 232 by analyzing an image (image information) captured by the imaging unit 800. In this case, the control device 900 may be configured to stop feeding at least one, preferably all, of the first fluid (hydrogel precursor), the second fluid (gelling agent), and the third fluid when an abnormality is detected.

Here, in a case where an abnormality of the first flow path 212, the second flow path 222, and/or the third flow path 232 is detected, the imaging unit 800 may be configured to capture an image of at least one of the first flow path 212, the second flow path 222, and the third flow path 232.

The abnormality of the hydrogel to be detected may be an abnormality of the size of the hydrogel, a significant variation in the shape of the hydrogel, and/or non-formation of the hydrogel. Abnormalities in the first flow path 212, the second flow path 222, and the third flow path 232 to be detected may be clogging of the flow path, flow disturbance, flow deviation on the same axis of the first fluid, the second fluid, and the third fluid, and the like.

In addition, the control device 900 may be configured to stop feeding at least one, preferably all, of the first fluid (hydrogel precursor), the second fluid (gelling agent), and the third fluid when detecting an abnormality in the pressure in at least one of the first flow path 212, the second flow path 222, and the third flow path 232, for example. The abnormality of the pressure can be detected by, for example, the pressure sensor 616 described above.

When the analysis unit 920 detects an abnormality of the hydrogel to be formed, the control device 900 may be configured to stop feeding the first fluid (hydrogel precursor), the second fluid (gelling agent), and/or the third fluid.

The display device 980 may be configured to be able to display a schematic view of at least one flow path, preferably a plurality of flow paths, more preferably all of the flow paths, of the first flow path 212, the second flow path 222, the third flow path 232, the first introduction path 132, the second introduction path 134, the third introduction path 136, and the cleaning flow path 138. Preferably, the display device 980 may display the state of each flow path together with each flow path.

### (Method for Producing Hydrogel)

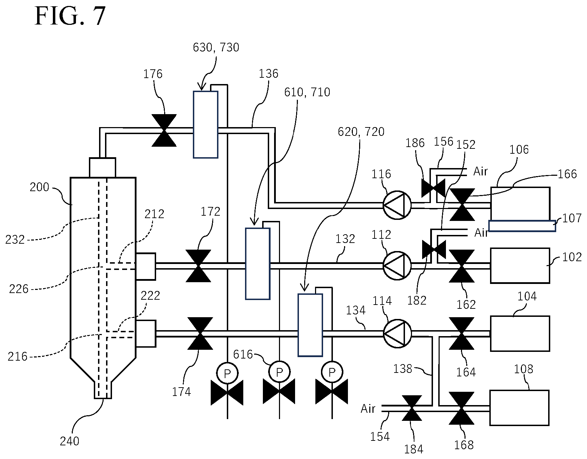

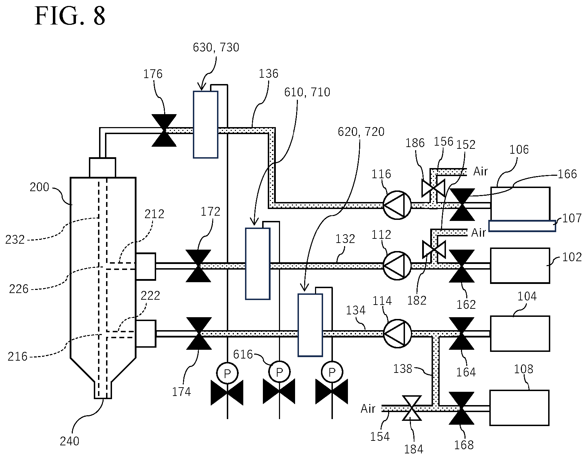

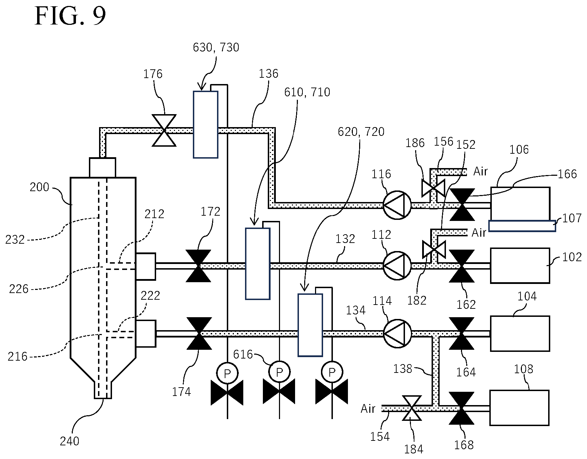

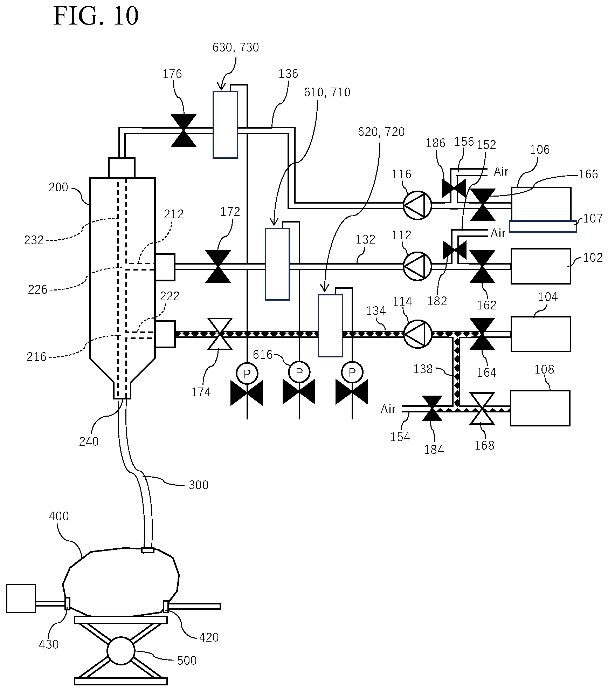

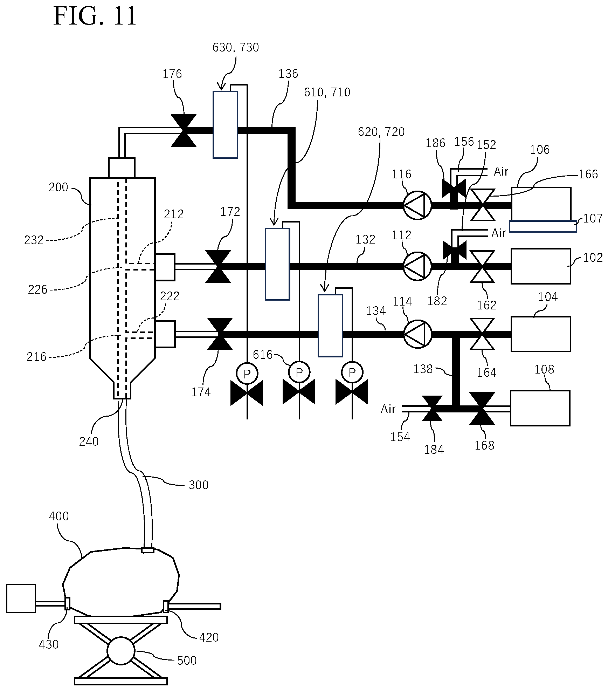

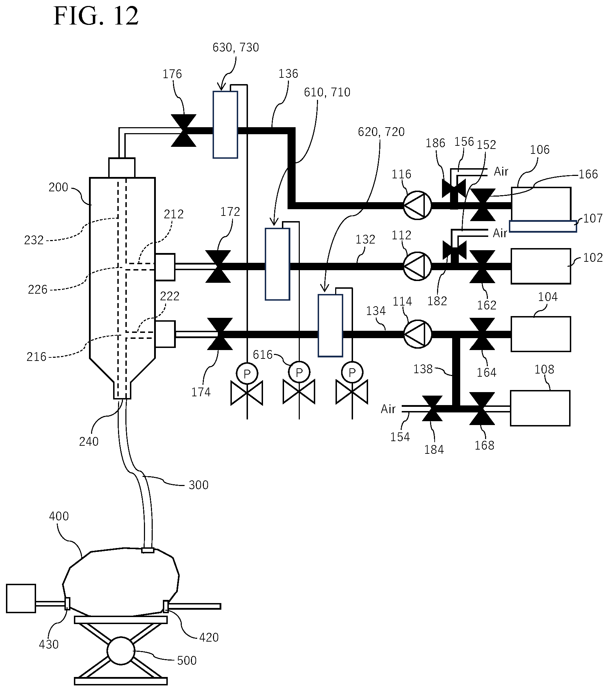

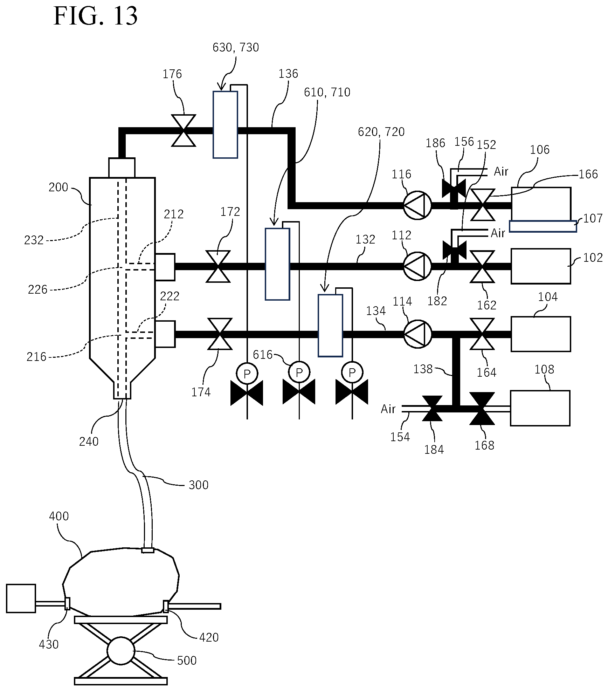

Next, a method for producing a hydrogel will be described with reference to Figs. 7 to 13. Fig. 7 is a schematic diagram illustrating a state in each introduction path at the initial stage of leak test. Fig. 8 is a schematic diagram for explaining the state in each introduction path in the subsequent stage following Fig. 7. Fig. 9 is a schematic diagram for explaining the state in each introduction path in the subsequent stage following Fig. 8. Fig. 10 is a schematic diagram for explaining the state in each introduction path in a pre-liquid feeding (pre-feeding) stage. Fig. 11 is a schematic diagram for explaining the state in each introduction path in the preparation stage of the pulsation suppressing unit and/or the defoaming unit. Fig. 12 is a schematic diagram for explaining the state in each introduction path in the subsequent stage following Fig. 11. Fig. 13 is a schematic diagram for explaining the state in each introduction path in the stage during the formation of the hydrogel.

### (1) Preparation

In the method for producing a hydrogel, first, the flow path forming member 200 is set in the housing 120. Specifically, the first flow path 212 of the flow path forming member 200 communicates with the first introduction path 132, the second flow path 222 of the flow path forming member 200 communicates with the second introduction path 134, and the third flow path 232 of the flow path forming member 200 communicates with the third introduction path 136.

In addition, the first fluid is introduced into the first storage section 102, the second fluid is introduced into the second storage section 104, and the third fluid is introduced into the third storage section 106. If necessary, a cleaning liquid is put into the cleaning container 108.

Here, the hydrogel flow path 300 and the storage container 400 need not be connected to the flow path forming member 200 yet. Instead, it is desirable that the outlet 240 side of the flow path forming member 200 be sealed.

### (2) Leak Test

Next, leakage inspection of the first introduction path 132, the second introduction path 134, and the third introduction path 136 is performed. Specifically, it is confirmed whether the first introduction path 132, the second introduction path 134, and the third introduction path 136 are airtight and/or liquid-tight. Alternatively or additionally, it is confirmed whether the flow path forming member 200 is connected to the first introduction path 132, the second introduction path 134, and the third introduction path 136 in an airtight and/or liquid-tight manner.

In the initial stage of the leak test, all the valves described above, that is, the first introduction valve 162, the second introduction valve 164, the third introduction valve 166, the fourth introduction valve 172, the fifth introduction valve 174, the sixth introduction valve 176, the cleaning valve 168, and the air valve 182, 184, and 186 may be in a closed state (see Fig. 7). Here, in Figs. 7 to 13, the drawings of the valves that are filled in black indicate that the valves are closed. On the other hand, the drawings of the valves that are not filled in black indicate that the valves are open.

Next, the air valves 182, 184, and 186 are opened, and the first pump 112, the second pump 114, and the third pump 116 are operated to introduce gas into the first introduction path 132, the second introduction path 134, and the third introduction path 136, and confirm whether there is a leak (see Fig. 8). Here, in Figs. 7 to 13, diagrams of flow paths and introduction paths painted in a dot shape indicate that gas is introduced.

More specifically, it is confirmed whether there is any leakage in the parts of the first introduction path 132, the second introduction path 134, and the third introduction path 136 that are on the upstream side of the fourth introduction valve 172, the fifth introduction valve 174, and the sixth introduction valve 176.

Next, the fourth introduction valve 172 is opened, and it is confirmed whether there is any leakage in the part on the downstream side of the fourth introduction valve 172 in the first introduction path 132 (see Fig. 9). Here, since the first introduction path 132 communicates with the second introduction path 134 and the third introduction path 136 via the flow path forming member 200, the fifth introduction valve 174 and the sixth introduction valve 176 may be closed. Thereby, it is possible to confirm in particular whether the first flow path 212 and the first introduction path 132 are communicated in an airtight and/or liquid-tight manner.

In addition, the fourth introduction valve 172 is closed and the fifth introduction valve 174 is opened, and it is confirmed whether there is any leakage on the downstream side of the fifth introduction valve 174 in the second introduction path 134. At this time, the fourth introduction valve 172 and the sixth introduction valve 176 may be closed. Thereby, it is possible to confirm in particular whether the second flow path 222 and the second introduction path 134 are communicated in an airtight and/or liquid-tight manner.

In addition, the fifth introduction valve 174 is closed and the sixth introduction valve 176 is opened, and it is confirmed whether there is any leakage on the downstream side of the sixth introduction valve 176 in the third introduction path 136. At this time, the fourth introduction valve 172 and the fifth introduction valve 174 may be closed. Thereby, it is possible to confirm in particular whether the third flow path 232 and the third introduction path 136 are communicated in an airtight and/or liquid-tight manner.

Here, the confirmation of the leakage of the first introduction path 132, the second introduction path 134, and the third introduction path 136 may be performed in any order. In addition, the occurrence of leakage at each introduction path can be detected by, for example, a pressure value or a pressure fluctuation value measured by the pressure sensor 616 connected to each introduction path.

When the leak test is finished, all the valves described above, that is, the first introduction valve 162, the second introduction valve 164, the third introduction valve 166, the fourth introduction valve 172, the fifth introduction valve 174, the sixth introduction valve 176, the cleaning valve 168, and the air valve 182, 184, and 186 may be closed.

### (3) Pre-Liquid Feeding (Pre-Feeding)

Next, the outlet 240 of the flow path forming member 200 and the storage container 400 are communicated in an airtight and/or liquid-tight manner via the hydrogel flow path 300. Then, the liquid is previously fed to the storage container 400 through the hydrogel flow path 300 (pre-feeding) .

In the pre-feeding, it is preferable that the cleaning liquid is fed to the storage container 400 through the outlet 240 of the flow path forming member 200 and the hydrogel flow path 300. Specifically, the cleaning liquid is sent by opening the cleaning valve 168 and the fifth introduction valve 174 and driving the second pump 114 (see Fig. 10). In Fig. 10, the flow paths in which a fill pattern is drawn represent the parts into which the cleaning liquid is being introduced.

The cleaning liquid reaches the outlet 240 through the cleaning flow path 138, the second introduction path 134, the second flow path 222, and the first junction point 216. Further, the cleaning liquid reaches the storage container 400 through the hydrogel flow path 300.

An object of the pre-feeding is to wet the inner surface of the tube constituting the hydrogel flow path 300. Thereby, it is possible to suppress the hydrogel formed in a later stage from adhering to the inner surface of the tube constituting the hydrogel flow path 300.

In the above embodiment, the cleaning liquid is fed to pass through the hydrogel flow path at the stage of pre-feeding. Alternatively, the fluid passing through the hydrogel flow path in the stage of the pre-feeding may be a liquid other than the cleaning liquid. For example, the fluid passing through the hydrogel flow path may be any one of the first fluid, the second fluid, and the third fluid. For the purpose of suppressing cell loss and reducing cost, the fluid passing through the hydrogel flow path is preferably a cleaning liquid or a second fluid.

When the pre-feeding is finished, all the valves described above, that is, the first introduction valve 162, the second introduction valve 164, the third introduction valve 166, the fourth introduction valve 172, the fifth introduction valve 174, the sixth introduction valve 176, the cleaning valve 168, and the air valve 182, 184, and 186 may be closed.

### (4) Preparation of Pulsation Suppressing Means and/or Defoaming Means

Preparation of the pulsation suppressing unit and/or the defoaming unit is performed as necessary, and may not be performed if unnecessary. In this preparation, the first introduction valve 162, the second introduction valve 164, and the third introduction valve 166 are opened, and the first pump 112, the second pump 114, and the third pump 116 is operated to feed the first fluid, the second fluid, and the third fluid (Fig. 11). The first fluid, the second fluid, and the third fluid are sent to the storage section 612 constituting the pulsation suppressing unit 610, 620, and 630 and/or the defoaming unit 710, 720, and 730, respectively. In Fig. 11, the flow paths in which a fill pattern is drawn represent the parts into which the first fluid, the second fluid, or the third fluid is being introduced.

When the first fluid, the second fluid, and the third fluid are sent to the storage section 612, the pressure in the storage section 612 increases. Next, as shown in Fig. 12, a valve provided in a part of the pressure sensor 616 is opened, and the pressure in the storage section 612 is adjusted to a desired value range (see Fig. 12). When this stage ends, the valve provided in the part of the pressure sensor 616 may be closed.

### (5) Stage During Hydrogel Formation

Next, a hydrogel is formed. The hydrogel production method using the hydrogel production apparatus includes flowing the third fluid toward the third flow path 232, flowing the first fluid (hydrogel precursor) to the first flow path 212, flowing the second fluid (gelling agent) to the second flow path 222, and joining the hydrogel precursor and the gelling agent at a first junction point 216 to form a hydrogel.

Specifically, the first introduction valve 162, the second introduction valve 164, the third introduction valve 166, the fourth introduction valve 172, the fifth introduction valve 174, and the sixth introduction valve 176 are opened, and the first pump 112, the second pump 114, and the third pump 116 are operated to feed the first fluid, the second fluid, and the third fluid (see Fig. 13). Preferably, the second operation unit 107 may be operated before the delivery of the third fluid to move the third fluid in the third storage section 106 (swing, vibration, or the like). When the third fluid contains suspended particles such as cells, the suspended particles in the third fluid can be uniformly dispersed by the action of the second operation unit 107.

Preferably, each of the first fluid, the second fluid, and the third fluid is continuously fed at a constant flow rate. As a result, a laminar flow in which the third fluid, the first fluid, and the second fluid are arranged in this order radially outward from the center is formed at the first junction point 216. In this state, the first fluid (hydrogel precursor) is gelled by the second fluid (gelling agent), whereby a tubular hydrogel containing the first fluid therein is formed (see Figs. 4 and 5). Here, the length of the tubular hydrogel can be adjusted by the feeding time of the first fluid, the second fluid, and the third fluid.

The hydrogel formed by the flow path forming member 200 is introduced into the storage container 400 through the hydrogel flow path 300.

### [Second Embodiment]

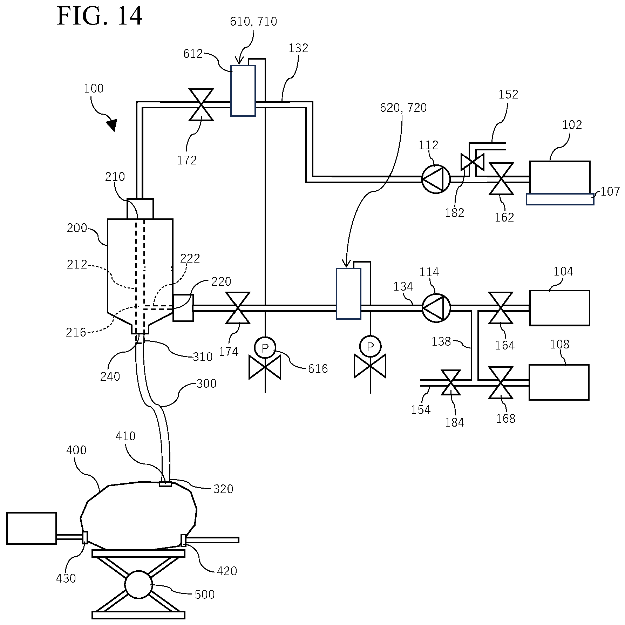

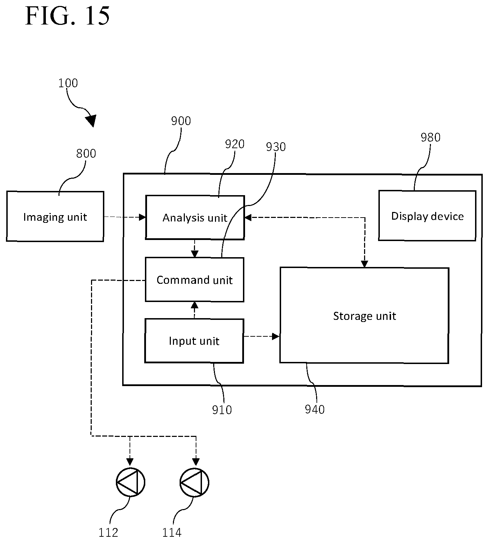

Fig. 14 is a schematic diagram for explaining the structure of a flow path of the hydrogel production apparatus according to the second embodiment. Fig. 15 is a block diagram of the hydrogel production apparatus according to the second embodiment. In the second embodiment, it should be noted that the description of the same configuration as that of the first embodiment may be omitted. In addition, the same reference numerals will be given to the same or corresponding configurations as those described in the first embodiment.

In the second embodiment, the hydrogel production apparatus 100 may include the first storage section 102, the second storage section 104, and the flow path forming member 200. The first storage section 102 stores a first fluid. The first fluid is a hydrogel precursor. Details of the hydrogel precursor are as described in the first embodiment.

The second storage section 104 stores the second fluid (gelling agent) that gelates the first fluid (hydrogel precursor) by coming into contact with the first fluid. Details of the gelling agent are as described in the first embodiment.

The flow path forming member 200 is a member including a first fluid flow path and a second fluid flow path. The flow path forming member 200 may have the first inlet 210, the second inlet 220, and the outlet 240. The first inlet 210 is an inlet for the first fluid. The second inlet 220 is an inlet for the second fluid. The flow path forming member 200 may have the first flow path 212 and the second flow path 222.

It should be noted that, in the second embodiment, the third storage section 106 or the third flow path described in the first embodiment are not provided.

The first flow path 212 is a flow path through which the hydrogel precursor flows, and may be defined by a flow path from the first inlet 210 to the outlet 240 via a first junction point 216 described later. The second flow path 222 may be defined by a flow path from the second inlet 220 to the first junction point 216.

The second flow path 222 is configured to join the second fluid (gelling agent) around the first fluid in the first flow path 212 at the first junction point 216. As a result, the second fluid flows along the flow direction of the first fluid around the flow of the first fluid. That is, the second fluid surrounds the first fluid (hydrogel precursor) in a cross section orthogonal to the flow of the first fluid.

The hydrogel production apparatus 100 may include a first pump 112 that feeds the first fluid and a second pump 114 that feeds the second fluid.

A hydrogel production method using the hydrogel production apparatus according to the second embodiment includes flowing the first fluid (hydrogel precursor) to the first flow path 212, flowing the second fluid (gelling agent) to the second flow path 222, and joining the hydrogel precursor and the gelling agent at the first junction point 216 to form a hydrogel.

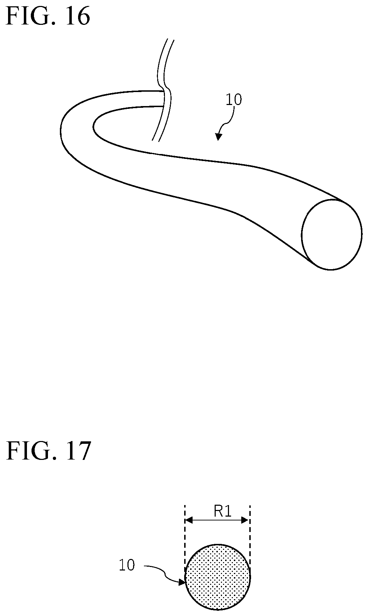

Preferably, each of the first fluid and the second fluid is continuously fed at a constant flow rate. As a result, a laminar flow in which the first fluid and the second fluid are arranged in this order radially outward from the center is formed at the first junction point 216. In this state, the first fluid (hydrogel precursor) is gelled by the second fluid (gelling agent), whereby a string-like hydrogel (solid hydrogel) is formed (see Figs. 16 and 17).

The hydrogel formed by the flow path forming member 200 is introduced into the storage container 400 through the hydrogel flow path 300.

Fig. 16 is a perspective view showing an example of the structure of the hydrogel formed in the second embodiment. Fig. 17 is a cross-sectional view of the hydrogel shown in Fig. 16. As shown in Fig. 16, the solid hydrogel 10 is formed in a long extending string shape.

The first fluid may contain cells. When the first fluid contains cells, the cells are embedded in the formed string-like hydrogel. In this case, the storage container 400 may be, for example, a culture container for culturing cells. The types of the cells are as described in the first embodiment.

The hydrogel production apparatus 100 may have the first pulsation suppressing unit 610 and/or the second pulsation suppressing unit 620. In addition, the hydrogel production apparatus 100 may include the first defoaming unit 710 and/or the second defoaming unit 720. The configurations of the first pulsation suppressing unit 610, the second pulsation suppressing unit 620, the first defoaming unit 710, and/or the second defoaming unit 720 are as described in the first embodiment.

In an example of the second embodiment, the input unit 910 may be configured to be capable of receiving set values of the flow velocity in the first flow path and the flow velocity in the second flow path. In other words, the user can input each of the flow velocity of the first fluid and the flow velocity of the second fluid from the input device. Alternatively, the input unit 910 may be configured to be able to receive one of the flow velocity in the first flow path and the flow velocity in the second flow path. The command unit 930 sends a command to operate the first pump 112 and the second pump 114 based on the input value received by the input unit 910.

Here, the flow velocity of the first fluid and the flow velocity of the second fluid affect a value related to the size of the string-like hydrogel to be formed. Specifically, the flow velocity V1 of the first fluid and the flow velocity V2 of the second fluid affect the diameter R1 of the string-like hydrogel to be formed. More specifically, the diameter R1 of the hydrogel increases as the ratio (V1/V2) of the flow velocity of the first fluid to the flow velocity of the second fluid increases.

Therefore, the user can appropriately adjust the diameter R1 of the string-like hydrogel to be formed by appropriately setting the flow velocity V1 of the first fluid and the flow velocity V2 of the second fluid.

The analysis unit 920 may calculate a value related to the size of the formed hydrogel. Specifically, the analysis unit 920 preferably calculates the diameter R1 of the formed hydrogel.

The storage unit 940 may store the results of the analysis by the analysis unit 920 in association with the value of the flow velocity set by the input unit 910. The value related to the size of the hydrogel calculated by the analysis unit 920, for example, the diameter R1 is preferably stored in the storage unit 940 in association with information related to the flow velocity V1 of the first fluid and the flow velocity V2 of the second fluid at the time of producing the hydrogel.

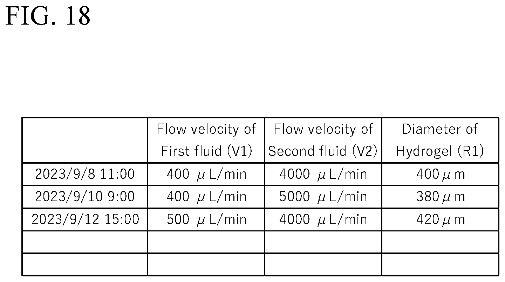

Fig. 18 is a diagram showing an example of data stored in the storage unit in the second embodiment. As shown in Fig. 18, the diameter R1 of the hydrogel calculated by the analysis unit 920 may be stored in the storage unit 940 in association with the flow velocity V1 of the first fluid and the flow velocity V2 of the second fluid at the time of producing the hydrogel.

As in the first embodiment, the input unit 910 may be configured to be able to receive information on the size of the hydrogel to be formed. For example, the input unit 910 may be configured to be able to receive the diameter R1 of the hydrogel to be formed.

When the control device 900 receives the information on the size of the hydrogel to be formed input by the user, for example, the diameter R1 of the hydrogel, the control device 900 automatically determines at least one of the flow velocity V1 of the first fluid and the flow velocity V2 of the second fluid based on the information on the size of the hydrogel. The flow velocity V1 of the first fluid and the flow velocity V2 of the second fluid can be acquired from, for example, information on the size of the hydrogel produced in the past. Specifically, the control device 900 can determine at least one of the flow velocity V1 of the first fluid and the flow velocity V2 of the second fluid based on the past data (see Fig. 18) stored in the storage unit 940, that is, the relationship between the flow velocity V1 of the first fluid, the flow velocity V2 of the second fluid, and the size (diameter R1) of the hydrogel. Here, the control device 900 may determine only one of the flow velocity V1 of the first fluid and the flow velocity V2 of the second fluid based on the information on the size of the hydrogel to be formed input by the user, for example, the diameter R1 of the hydrogel, and the user may determine the other one of the flow velocity V1 of the first fluid and the flow velocity V2 of the second fluid.

The control device 900 may be configured to control (feedback control) the flow velocity of at least one of the first fluid (hydrogel precursor) and the second fluid (gelling agent) based on the results of the analysis by the analysis unit 920. Specifically, as described above, the analysis unit 920 calculates the diameter R1 of the hydrogel actually formed based on the image of the hydrogel formed. Further, the analysis unit 920 calculates a deviation of the diameter R1 of the hydrogel actually formed from the set value of the diameter of the hydrogel received by the input unit 910. The command unit 930 may control, that is, change the flow velocity of at least one of the first fluid (hydrogel precursor) and the second fluid (gelling agent) to eliminate the deviation.

As described above, the diameter R1 of the hydrogel increases as the ratio (V1/V2) of the flow velocity of the first fluid to the flow velocity of the second fluid increases. Based on this relationship, the control device 900 may change the flow velocity of at least one of the first fluid and the second fluid such that the size of the hydrogel actually formed becomes close to the value of the size of the hydrogel input by the user.

The method for producing a hydrogel in the second embodiment can be carried out substantially in the same manner as the production method described in the first embodiment. However, in the second embodiment, since the third flow path and the configuration associated therewith are not provided, among the steps described in the production method described in the first embodiment, steps associated with the third flow path and the configuration associated therewith may be omitted.

As described above, the contents of the present invention have been disclosed through the embodiments, but it should not be understood that the description and the drawings constituting a part of the disclosure limit the present invention. From this disclosure, various alternative embodiments, examples, and operational techniques will become apparent to those skilled in the art. Therefore, the technical scope of the present invention is defined only by the matters specifying the invention according to the claims appropriate from the above description.

This application claims priority based on Japanese Patent Application No. 2023-175600 filed on October 10, 2023, the entire contents of which are incorporated herein by reference.

## Claims

1. A hydrogel production apparatus comprising:
a first flow path for flowing a hydrogel precursor;
a second flow path for flowing a gelling agent that gels the hydrogel precursor;
a first junction point of the first flow path and the second flow path;
a storage container containing a hydrogel that is formed by contact between the hydrogel precursor and the gelling agent; and
a hydrogel flow path that communicates in a closed manner at least between the storage container and the first junction point.

2. The hydrogel production apparatus according to claim 1, wherein the storage container is constituted by a flexible container having an expandable and contractible internal space.

3. The hydrogel production apparatus according to claim 1 or 2, wherein the hydrogel flow path is constituted by a flexible tube.

4. The hydrogel production apparatus according to any one of claims 1 to 3, comprising a first operation unit that moves the liquid in the storage container.

5. The hydrogel production apparatus according to any one of claims 1 to 4, wherein the storage container is a culture container for culturing cells.

6. The hydrogel production apparatus according to any one of claims 1 to 5, comprising pulsation suppressing unit for suppressing pulsation of the hydrogel precursor and/or the gelling agent on an upstream side of the first junction point.

7. The hydrogel production apparatus according to any one of claims 1 to 6, comprising defoaming unit for removing bubbles in a solution containing the hydrogel precursor and/or the gelling agent on the upstream side of the first junction point.

8. The hydrogel production apparatus according to claim 7, wherein the defoaming unit is located between a pump and the first junction point, and has a storage section capable of holding gas.

9. The hydrogel production apparatus according to any one of claims 1 to 8, comprising
a pump for flowing the hydrogel precursor and/or the gelling agent,
wherein the pump has a performance such that, excluding periods of startup and shutdown, at a specific average flow velocity value within the range of 0.1 mL to 1000 mL/min, the variation in flow velocity remains within 20% of the specified average flow velocity value.

10. The hydrogel production apparatus according to any one of claims 1 to 9, comprising an imaging unit that acquires a form of the hydrogel on a downstream side of the first junction point or at the first junction point.

11. The hydrogel production apparatus according to claim 10, comprising:
an analysis unit that analyzes the form of the hydrogel imaged by the imaging unit, and
a control device that controls a flow velocity of at least one of the hydrogel precursor and the gelling agent based on results of analysis by the analysis unit.

12. The hydrogel production apparatus according to claim 11, wherein
the analysis unit is configured to calculate a size of the formed hydrogel, and
the control device is configured to control the flow velocity of at least one of the hydrogel precursor and the gelling agent based on the size of the hydrogel calculated by the analysis unit.

13. The hydrogel production apparatus according to claim 11 or 12, comprising:
an input unit that sets the flow velocity of at least one of the hydrogel precursor and the gelling agent, and
a storage unit that stores results of analysis by the analysis unit together with the value of the flow velocity set by the input unit.

14. The hydrogel production apparatus according to any one of claims 1 to 13, comprising
a third flow path for flowing a fluid,
wherein the third flow path is configured to join the first flow path at a second junction point located on the upstream side of the first junction point.

15. The hydrogel production apparatus according to claim 14, comprising:
a third storage section for storing the fluid, and
a second operation unit for moving a fluid in the third storage section.

16. The hydrogel production apparatus according to claim 15, comprising:
an imaging unit that acquires a form of the hydrogel on a downstream side of the first junction point or at the first junction point,
an analysis unit that analyzes the form of the hydrogel imaged by the imaging unit, and
a control device that controls the flow velocities of at least one of the hydrogel precursor, the gelling agent, and the fluid based on the results of analysis by the analysis unit.

17. The hydrogel production apparatus according to claim 16, wherein
the fluid contains cells, and
the analysis unit is configured to estimate the cell density in the hydrogel based on image information captured by the imaging unit.

18. The hydrogel production apparatus according to any one of claims 15 to 17, comprising:
an input unit that sets the flow velocities of at least one of the hydrogel precursor, the gelling agent, and the fluid, and
a storage unit that stores results of analysis by the analysis unit together with the value of the flow velocity set by the input unit.

19. The hydrogel production apparatus according to any one of claims 11 to 13 and 15 to 18, wherein
the analysis unit detects an abnormality in at least one of the hydrogel, the first flow path, and the second flow path, and
the control device is configured to stop the liquid feeding of the hydrogel precursor and the gelling agent when the abnormality is detected.

20. The hydrogel production apparatus according to any one of claims 1 to 19, comprising
a flow path forming member in which the first flow path and the second flow path are formed,
wherein the flow path forming member has an outlet through which the hydrogel formed by the hydrogel precursor and the gelling agent flows out, and
a distance from the first junction point to the outlet is in the range of 3 mm to 150 mm.

21. The hydrogel production apparatus according to any one of claims 1 to 20, wherein the hydrogel flow path is constituted by a rigid tube.

22. A hydrogel production method using the hydrogel production apparatus according to any one of claims 1 to 21, the method comprising:
flowing the hydrogel precursor into the first flow path;
flowing the gelling agent into the second flow path;
joining the hydrogel precursor and the gelling agent at the first junction point to form a hydrogel; and
introducing the hydrogel into the storage container through the hydrogel flow path.

23. The hydrogel production method according to claim 22, wherein
the storage container is constituted by a flexible container having an expandable and contractible internal space, and
the internal space of the storage container is set in an expandable state before the liquid feeding of the hydrogel precursor and the gelling agent.

24. The hydrogel production method according to claim 22 or 23, further comprising performing pre-feeding in which a liquid is passed through the hydrogel flow path before the formation of the hydrogel.

25. The hydrogel production method according to any one of claims 22 to 24, further comprising inspecting whether there is any leakage in the flow paths included in the hydrogel production apparatus before the formation of the hydrogel.
